Europäisches Patentamt

European Patent Office

Office européen des brevets

(19)

(11) EP 1 479 399 A1

(12) EUROPEAN PATENT APPLICATION

(43) Date of publication:
24.11.2004 Bulletin 2004/48

(51) Int Cl.⁷: A61K 51/12, A61K 51/06, G21G 4/08

(21) Application number: 03101480.6

(22) Date of filing: 22.05.2003

(84) Designated Contracting States:
AT BE BG CH CY CZ DE DK EE ES FI FR GB GR HU IE IT LI LU MC NL PT RO SE SI SK TR
Designated Extension States:
AL LT LV MK

(71) Applicant: EUROPEAN ATOMIC ENERGY COMMUNITY (EURATOM)
1049 Brussels (BE)

(72) Inventors:
• Apostolidis, Christos
69115 Heidelberg (DE)
• Brandalise, Bruno
76351 Linkenheim (DE)

• Carlos-Marquez, Ramon
76227 Karlsruhe-Durlach (DE)
• Janssens, Willem
21020 Ranco (IT)
• Molinet, Roger
76351 Linkenheim (DE)
• Nikula, Tuomo
76297 Stutensee (DE)

(74) Representative: Schmitt, Armand et al
Office Ernest T. Freylinger S.A.
234, route d'Arlon,
B.P. 48
8001 Strassen (LU)

(54) **Method of loading a radioelement generator with mother radionuclide**

(57) The invention relates to a method of loading a mother radionuclide into a radionuclide generator which comprises an cation exchange material, the said method comprising a preliminary step which consists in passing over the said material a solution containing the mother radionuclide and a complexing agent for the said radionuclide, the said complexing agent being at an effective concentration so as to prevent the mother radionuclide from concentrating solely in one part of the volume of the cation exchange material, the cation exchange material being chosen in the group consisting of the macroporous resins, the resins having a crosslinking rate of from 1 to 12%.

This method is applied to radioisotope generators which emit α particles and are used in particular in the field of radioimmunotherapy.

**Description**

**TECHNICAL FIELD**

The object of the present invention is to provide a method of loading a radionuclide generator with a mother radionuclide, in particular a radionuclide generator where at least one of the radionuclides in cascade emits $\alpha$ particles, such as a $^{225}$Ac/$^{213}$Bi generator.

The field of the invention may therefore be defined as that of radionuclide generators from which can be eluted $\alpha$ particles emitting radionuclides, the said radionuclides being suitable for use in particular in the field of radioimmunotherapy.

Generally speaking, radioimmunotherapy consists in carrying radioisotopes via transporters such as monoclonal antibodies towards malignant cells or groups of such cells. In the course of their radioactive decay, these radioisotopes release energetic particles which are capable of destroying the malignant cells. In the past, this technique have used radionuclides which during their decay emit $\beta$ particles.

These particles are long-distance particles (of the order of 1 to 10 mm) which, consequently, have the capacity to destroy a group of cells rather than isolated cells and, consequently, are effective in the treatment of large tumours. In recent years, research also concentrated on radionuclides and radionuclide generators which emit $\alpha$ particles.

The reason for this is that these particles, owing to their short distances (of the order of the diameter of a limited number of cells) and their very high linear energy transfer, are particularly suitable for the targeted destruction of micrometastases or else of isolated malignant cells, as in the case of leukaemia.

Moreover, the cells irradiated by the $\alpha$ particles have a reduced capacity for repairing the damaged DNA, so rendering these particles of great interest for the destruction of isolated malignant cells.

Generally speaking, the production for therapeutic purposes of radionuclides which emit $\alpha$ particles is carried out using generators of such radionuclides.

In the majority of cases, these generators are present in the form of at least one column packed with a material on which a radionuclide, referred to as the mother radionuclide, is absorbed in a loading operation. This mother radionuclide, in the course of its radioactive decay, generates various radionuclides which emit $\alpha$ particles (and are called daughter radionuclides), which are separated from the mother radionuclide during one or more elution steps.

In order to be able to be used for therapeutic purposes, these daughter radioisotopes may be fixed directly, by various techniques, onto monoclonal antibodies or other carriers (biomolecules) which are capable of targeting a group of cells to be treated with the $\alpha$ particles.

By way of examples of daughter radionuclides which are emitters of $\alpha$ particles, mention may be made of $^{211}$At ($t_{\frac{1}{2}}$ = 7.2 h), $^{212}$Bi ($t_{\frac{1}{2}}$ = 60.6 min), $^{213}$Bi ($t_{\frac{1}{2}}$ = 45.6 min), $^{223}$Ra ($t_{\frac{1}{2}}$ = 11 days) and $^{255}$Fm ($t_{\frac{1}{2}}$ = 7.2 h).

Greater details concerning the radionuclides which emit $\alpha$ particles are available in the publication by Mirzadeh, "Generator-produced Alpha-emitters", Appl. Radiat. Isot., Vol. 49, No. 4, pp. 345-349, 1998 [1] and the publication by McDevitt et al., "Radioimmunotherapy with alpha-emitting nuclides", European Journal of Nuclear Medicine, Vol. 25, No. 9, September 1998, pp. 1341-1351 [2].

Numerous articles in the literature describe $\alpha$ radionuclide generators, especially generators which produce $^{212}$Bi and $^{213}$Bi (the mother radionuclides being, respectively, $^{228}$Th or $^{224}$Ra and $^{225}$Ac).

Accordingly, the authors Zucchini et al. in "Isotopic Generator for $^{212}$Pb and $^{212}$Bi", Int. J. Nucl. Med. Biol., Vol. 9, pages 83-84, 1982 [3], the authors Atcher et al., "An improved Generator for the Production of $^{212}$Pb and $^{212}$Bi from $^{224}$Ra", Appl. Radiat. Isot., Vol. 39, No. 4, pp. 283-286, 1988 [4], and the authors Geerlings et al., "The Feasibility of $^{225}$Ac as a source of $\alpha$-particles in radioimmunotherapy", Nuclear Medicine Communications (1993), 14, pp. 121-125 [5], describe generators which consist of a column packed with an organic or inorganic material on which the mother radionuclides are absorbed.

However, the drawback of the generators described in the above-mentioned publications is that they are subject to premature degradation of the materials packing the column.

In effect, during the step of loading the column with mother radionuclide, such as $^{225}$Ac, the said nuclide is absorbed primarily, by affinity with the material packing the column, at the top of the said column. Consequently, the material at the top of the column is exposed to intense irradiation owing to the local emission of $\alpha$ particles, so causing it to degrade.

In order to resolve this phenomenon of localized radiolysis, the authors Wu et al. in "An improved Generator for the Production of $^{213}$Bi from $^{225}$Ac", Radiochimica Acta 79, pp. 141-144 (1997) [6] propose distributing the mother radionuclide over the column by means of the following loading method, comprising the following successive steps:

- packing a column with a resin having a high affinity for $^{225}$Ac, the said resin being subsequently equilibrated with a nitric acid solution;

- withdrawing approximately (2/3) of the resin packing the column, the said withdrawn resin being subsequently transferred to a test tube containing the mother radionuclide $^{225}$Ac and the resulting mixture being incubated at ambient temperature and subjected to vigorous stirring; and

- returning the resin impregnated with mother radionuclide to the initial column.

Despite the fact that this loading method, set out above, allows the radionuclide to be distributed over the major part of the column, it has the important drawback of giving rise to contamination of the exterior during its implementation, and is also difficult to realize on a large scale. The method also has a risk to expose the workers to high radiation doses.

Patent Application EP 0967618 A1 [7] likewise describes a method of loading a $^{225}$Ac radionuclide generator, the said method aiming to minimize the phenomenon of localized radiolysis by using a continuous circulation of eluent during the storing step.

More specifically, a continuous eluent circulation of the daughter radionuclide $^{213}$Bi is provided over the entire length of the column. This method has the drawback of necessitating the installation of a unit for eluent circulation of the daughter radionuclide during the storing of the generator, thereby ruling out the implementation of this method on a large scale. The circulation also increase actinium-225 breakthrough.

To summarize, the prior art methods of loading a generator with mother radionuclide all feature one or more of the following drawbacks:

- they give rise to localized radiolysis of the generator, owing to concentration of the radionuclide at the top of the generator;

- they give rise to pollution outside the generator, during the packing of the column;

- they are not suitable for large scale application.

The aim of the present invention is to provide a method of loading a generator of radionuclides with a mother radionuclide, the said method being devoid of the aforementioned drawbacks and, in particular, allowing homogenous distribution of the said radionuclide in the optimal volume of the generator, and allowing the minimisation of the risks of the breakthrough of the mother radionuclide during the elution steps.

This aim is achieved by means of a method of loading a mother radionuclide into a radionuclide generator which comprises a cation exchange material, the said method comprising a preliminary step which consists in passing over the said material a solution containing the mother radionuclide and a complexing agent for the said mother radionuclide, the said complexing agent being at an effective concentration such as to prevent the mother radionuclide from concentrating solely in one part of the volume of the cation exchange material, the cation exchange material being chosen in the group consisting of the macroporous resins, the resins having a crosslinking rate of 1% to 12%.

Advantageously, the passing of a solution comprising the mother radionuclide and a complexing agent for the said radionuclide thus makes it possible to obtain a distribution of the radionuclide (in complexed form) over the optimal volume of cation exchange material. The reason for this is that the complex formed by the radionuclide and its complexing agent exhibits the capacity to be fixed to the cation exchange material to a lesser extent than the weakly or uncomplexed radionuclide. Consequently, the mother radionuclide thus complexed no longer has a tendency to concentrate in any given part of the material. There is therefore a difference between this and the prior art embodiments where the generator was packed with a mother radionuclide by passing in a solution containing solely the mother radionuclide in the environment, where required amount and type of the mother radionuclide - complexing agent complex is not present.

In the embodiments of the prior art, the mother radionuclide, owing to its strong affinity with the material constituting the generator, became concentrated at the site where the solution was introduced, and contributed to the deterioration of the generator at the site of its concentration, owing to an intense radiative emission generated by the radioactive decay of the mother radionuclide.

Furthermore, the authors have discovered that, by using a cation exchange material as mentioned above, i. e macroporous resins and resins having a crosslinking rate of 1% to 12%, the both having a high exclusion limit, it is possible to get better distribution of the mother radionuclide over column without significant decrease of amount of the daughter radionuclide(s) in the eluant.

The said resins may be, for example, a styrenedivinylbenzene or styrene-acrylic acid copolymer with exchanger groups such as $SO_3^-$ groups.

The exclusion limit of a support is defined by the size of the largest molecule able to enter the pores under a given set of conditions. Typically at ionexchange resins which have crosslinking rate decrease from 12% to 1% the exclusion limit increase from roughly 400 to 3500 the higher exclusion limits are concerned to be macroporous resins.

Several companies are producing this type resin with different trade names for example Bio-Rad has at least following resin which can be suitable to this application:

AG50W resin (available with different crosslinking rate)
AG MP50 resin (macroporous resin)

The selection of the complexing agent effective for meeting the conditions would of course be made as a function of the mother radionuclide which is incorporated into the generator. The effective concentration of a considered complexing agent would be made as a function of the resin used in the generator.

Some examples of complexant agents can be acetate ion, $Cl^-$ (other halogen ions are also suitable), $NO_3^-$.

Once the complexing agent has been selected as a function of the nature of the radionuclide, this complexing agent, for a given cation exchange material, must be present in the solution at a concentration which is effective to prevent a concentration of the said radionuclide at a specific site in the material. This concentration may easily be determined by the person skilled in the art in the course of experiments. For example, the selection of an effective concentration may be made by determining, for solutions at different concentrations, the radioactivity which is not bound on the cation exchange material (the activity being expressed as a percentage of total activity).

Preferably, the complexing agent must be at a concentration which is effective to give a distribution coefficient $K_D$ between the solution and the cation exchange material from 0.5 to 0.99, preferably from 0.7 to 0.98.

More specifically, this distribution coefficient in the mother radionuclide between the solution and the cation exchange material corresponds to the following ratio:

(concentration of mother radionuclide over the

material)/(total mother radionuclide concentration).

In practice, this coefficient is determined by batch experiments as follows:

- the cation exchange material is mixed with a solution containing the mother radionuclide and a complexing agent for the said radionuclide;

- the mixture is incubated and then centrifuged;

- the concentration of the radionuclide in the material is determined by various techniques, such as gamma spectroscopy, and then the ratio of the concentration of radionuclide on the cation-exchange material to the total radionuclide concentration is calculated (this concentration being known from the start).

The method of loading, according to the present invention, may further comprise at least one step of washing, which consists, after the preliminary step mentioned above, in passing over the cation exchange material a solution containing at least one complexing agent for the mother radionuclide. This step thus makes it possible to improve still further the distribution of the mother radionuclide over the cation exchange material. Note that the complexing agent used for washing may be identical to or different from that used during the preliminary step of the method of the invention. This washing solution has advantageously a significant lower concentration of complexing agent than the solution which is used in the preliminary step or other substances to prevent hydrolyse of the metal ion, in order to prevent the phenomenon of breakthrough of the radionuclide during washing.

The method of loading, according to the invention, may advantageously comprise, after the preliminary step or the optional washing steps, at least one step which consists in passing over the said cation exchange material a solution containing an agent capable of destroying the complex formed between the mother radionuclide and its complexing agent.

In other words, when the mother radionuclide has been distributed in the form of a complex over the optimised part of the volume of the material, by virtue of the preliminary step of the method and of the optional washing steps, the complexed radionuclide may advantageously undergo decomplexation. When thus decomplexed, the mother radionuclide becomes strongly fixed to the cation exchange material, thereby preventing this radionuclide undergoing breakthrough during the steps of elution of the daughter radionuclides.

Decomplexation agent is all substances which destroy effectively the complex between radionuclide and complex agent including, for example, an effective concentration of original complex agent allowing the radionuclide to retain on the cation exchange material.

As a decomplexing solution it is possible for example to use a solution containing a minimal amount of a complexing agent (identical to or different from that used during the preliminary step) to allow to break down the complex between metal ion and a complexing agent which was used in the course of the preliminary step, this minimal amount being determined by a man skilled in the art. Consequently, the simple fact of adding a small quantity of this new complexing agent to the solution to be passed over the material will contribute to destroying the complex formed during the preliminary step of the method. The radionuclide thus liberated is absorbed directly on the cation exchange material, owing to the fact that it has an even higher affinity for the cation exchange material of the generator than for the complexing agent of the decomplexing solution.

Explicitly, this phenomenon of decomplexation may be illustrated by the following reactions:

$$\text{Complex 1} + R_{mother} \quad \rightleftharpoons \quad \text{Complex 1} - R_{mother} \quad (1)$$

$$\text{Complex 1} + R_{mother} \quad \xrightarrow{+ \text{ decomplexing agent 2}} \quad \text{Complex 1} - R_{mother} \quad (2)$$

$$\updownarrow$$

$$R_{mother}\text{-material}$$

Complex 1: complex used during the preliminary step of the method;

$R_{mother}$: mother radionuclide;

Material: cation exchange material which makes up the generator.

Equation (1) illustrates the complexation equilibrium which exists in the solution employed in the course of the preliminary step of the method, this equilibrium being largely shifted in the direction of formation of the complex.

Equation (2) illustrates the equilibrium during decomplexion shifting to right in the course of the decomplexation. Once decomplexed, the mother radionuclide is absorbed on the material, owing to the fact that the forces of attraction of the material are greater than the forces of complexation of the decomplexing agent 2.

The method of loading, according to the present invention, may be applied to any type of radioelement generator, especially radioelement generators which emit $\alpha$ particles.

This method therefore most particularly finds application for the loading of generators in which the mother radionuclide is $^{225}$Ac, this mother radionuclide producing, during its radioactive decay, several daughter radionuclides which are emitting $\alpha$ particles.

In the case of a radionuclide generator whose mother radionuclide is $^{225}$Ac, the cation exchange material of the generator may be a macroporous resin containing $-SO_3^-$ groups as exchanger groups. This material may in particular be a resin based on styrene and divinylbenzene, bearing $SO_3^-$ ionic groups.

A particularly effective complexing agent for $^{225}$Ac may preferably be selected from nitric acid, hydrochloric acid and mixtures thereof.

In particular, when the complexing agent selected to complex $^{225}$Ac is nitric acid, particularly when the cation exchange material is a resin based on styrenedivinylbenzene bearing $-SO_3^-$ group, it may advantageously be used, in the solution containing $^{225}$Ac and the said complexing agent, at concentrations greater than or equal to 1.5 $mol.l^{-1}$, preferably from 2 to 2.5 $mol.l^{-1}$, when used high crosslinked resins such as resins having a crosslinking rate over 5 to 8% (Higher concentrations are useful when used less crosslinked materials)and preferably from 5 to 7.5 mol.$l^{-1}$ when used macroporous resins.

The optional washing step to get more uniformly distribution may be effected by passing through the cation exchange material a solution of nitric acid at the same as or lower concentration than the concentration used in the preliminary step.

When the complexing agent is $HNO_3$, the said decomplexation being intended to fix $^{225}Ac$ on the column and to minimize the phenomenon of breakthrough of the $^{225}Ac$ outside the column, may advantageously be carried out by passing through the cation exchange material at least once a solution of hydrochloric acid having, for example, a concentration of from 0.001 to 0.01 mol.$l^{-1}$.

When the complexing agent is $HNO_3$, the said decomplexation being intended to fix $^{225}Ac$ on the column and to minimize the phenomenon of breakthrough of the $^{225}Ac$ outside the column, may advantageously be carried out by passing through the cation exchange material at least once a solution of nitric acid having, for example, a concentration of from 0.001 to 0.01 mol.$l^{-1}$.

As the complexing agent for the actinium-225 it is also possible to use hydrochloric acid, which may be used advantageously, in the solution containing $^{225}Ac$, at concentrations of more than 2 mol.$l^{-1}$, preferably from 2,5 to 3.5 mol.$l^{-1}$ when used high crosslinked resins, such as resins having a crosslinking rate over 5 to 8%. (Higher concentrations are useful when used less crosslinked materials).

The optional washing step to get more uniformly distribution may be effected by passing through resin a solution of hydrocloric acid at the same or lower concentration, than the concentration used in the preliminary step.

In detail, the method of loading a generator with mother radionuclide $^{225}Ac$ may be carried out in the manner which is set out hereinbelow.

Thus, a solution containing the mother radionuclide and its complexing agent is passed through the cation exchange material which makes up the column (by simple gravity or peristaltic pump (any method which allow the solution to pass the column)).

Note that prior to the method of loading with actinium-225, the generator is generally prepared by packing a column, for example, in glass, polyethylene (or any other suitable material) tube with the required supporting material to hold resin in the tube with an appropriate cation exchange material. Once prepared, this column, before the passage of the solution containing the mother radionuclide and its complexing agent, may be equilibrated by passing over the cation exchange material a solution containing solely the complexing agent of the mother radionuclide.

In order to improve the distribution of the mother radionuclide over the cation exchange material, the loading method may include one or more steps of washing the material with a solution containing solely the complexing agent present in the solution containing the radionuclide or optionally containing another complexing agent for the radionuclide.

Finally, in order to minimize breakthrough, the loading method of the present invention may comprise a step of decomplexing the said mother radionuclide, the said step consisting in passing over the cation exchange material a solution containing an agent capable of liberating the mother radionuclide from its complexed form. On account of the high affinity of the cation exchange material for the mother radionuclide in decomplexed form, the radionuclide thus decomplexed is instantaneously fixed to the cation exchange material of the generator.

Note that, once the loading method, according to the invention, has been implemented, it is followed conventionally by one or more steps of elution which are aimed at the daughter radionuclide or radionuclides. When the daughter radionuclides have been recovered in an elution solution, the said solution may be used, in the course of a subsequent step, for the labelling of antibodies, monoclonal antibodies for example, or other biomolecules with a view to transporting the said radionuclides to specific sites. The invention therefore finds its application in the field of radio (immuno)therapy.

The invention will now be described in more detail with reference to the examples which are set out hereinbelow.

## DETAILED EXPOSITION OF THE INVENTION

The examples which follow illustrate the efficiency of the inventive $^{225}Ac$ loading method, particularly using different cation exchange materials with different $^{225}Ac$-complexing agents at different concentrations.

For each of the examples set out below, the actinium-225 is obtained from a $^{229}Th$ source and is purified to remove any $^{225}Ra$ contamination.

Greater details relating to obtaining a pure solution of $^{225}Ac$ are available in the publication by Apostolidis et al., "Production of carrier-free actinium-225/bismuth 213 from thorium-229 for alpha-immunotherapy", J. Labelled Cpd. Radiopharm.44, Suppl. 1 pp. 806-808 [8]. When the pure actinium-225 solution has been obtained, this solution is evaporated to dryness and the residue is subsequently dissolved in a small volume of an acidic solution, which is the same solution that this used to complex the mother radionuclide.

The generators which are used in the context of these examples are in the form of a column of plastic material Polyethylen (PE) . The column, before loading of the mother radionuclide, is packed with a cation exchange material, to give an ion exchange column measuring 0.4 4 * 4 cm (diameter * height) and having a total volume of 0.5 ml. This column is subsequently equilibrated by passing through the said column a solution containing solely a complexing agent for the mother radionuclide.

The pure solution of actinium-225 is subsequently diluted by adding a volume of complexing agent solution, the said volume being equivalent to twice that of the column prepared above.

The generators are subsequently loaded with this solution.

In order to homogenize the actinium distribution, in each of the experiments washing is carried out using a solution containing solely the complexing agent which the same concentration as the initial loading solution, the said washing solution having a volume equivalent to 6 times that of the column (approximately 3ml).

EXAMPLE 1

In this example, the cation exchange material used is a macroporous resin (AGMP 50 BIORAD) exclusion limit >1 000 000 based on styrene and divinylbenzene which contains ionic groups of $-SO_3^-$ type. The particle size was 100-200 mesh (75-150 micrometer)

The column is loaded with $^{225}$Ac by passing through the said column, in the course of a preliminary step, a solution containing $^{225}$Ac 1 mCi (37 MBq) and nitric acid at different concentrations (from 4 to 10 mol.$1^{-1}$), this solution having a volume corresponding to twice the volume of the column.

For this example, the process of loading with actinium-225 also comprises washing with six bed volumes with the same nitric acid concentration, which has been used during the initial loading. The decomplexation step comprises washing with two bed volumes of 0.005 M $HNO_3$. To convert column from nitrate form to chloride form the generator was washed with 1 ml of 2 M HCl. Finally the column was stabilised with 0.001-0.01 M HCl

In order to demonstrate the efficiency of the loading method according to the invention, measurements were made, at different sites in the column, of the % radio activity released at these sites, relative to the total activity (the measurement of the activity corresponding to an indirect measurement of the distribution of actinium in the column).

The results are given in Table 1 below:

TABLE 1

| Site in column (top to bottom) | $HNO_3$ 4 M | $HNO_3$ 5 M | $HNO_3$ 6 M | $HNO_3$ 6.5 M | $HNO_3$ 7 M | $HNO_3$ 7.5 M | $HNO_3$ 8 M | $HNO_3$ 9 M | $HNO_3$ 10 M |
|---|---|---|---|---|---|---|---|---|---|
| 1st quarter | 100 | 99 | 82 | 29 | 23 | 15 | 11 | 4 | 4 |
| 2nd quarter | - | 1 | 19 | 60 | 66 | 64 | 34 | 20 | 23 |
| 3rd quarter | - | - | - | 11 | 11 | 17 | 47 | 50 | 42 |
| 4th quarter | - | - | - | - | - | 4 | 7 | 22 | 25 |

"1st quarter", "2nd quarter", and so on means the 1st, 2nd, etc. quarter of volume starting from the top of the column, in other words the zone where the solutions are introduced and the Ac-225 distributed.

In this example, therefore, the distribution which is observed of the actinium-225 over the volume of the column is entirely satisfactory (in other words, an actinium distribution which extends over an optimal of the volume of the column and no longer at the top of the column as is the case for the loading embodiments of the prior art) for nitric acid concentrations from 6 to 10 mol.$1^{-1}$. The most optimal concentration is 6.5 to 7 mol.$l^{-1}$.

EXAMPLE 2

In this example, the procedure followed in Example 1 is repeated but using, as cation exchange material, a highly cationic resin based on styrene and divinylbenzene at 8% crosslinked resin (Dowex 50 Wx8, FLUKA). The particle size was 100-200 mesh (75-150 micrometer), containing $-SO_3^-$ groups as ionic groups, and a complexing

solution $HNO_3$ having concentrations of from 1.0 to 3 $mol.l^{-1}$. For this example, the process of loading with actinium-225 also comprises washing with six bed volumes with the same nitric acid concentration, which has been used during the initial loading. The decomplexation step comprises washing with two bed volumes of 0.005 M $HNO_3$. To convert column from nitrate form to chloride form the generator was washed with 2 M HCl. Finally the column was stabilised with 0.001-0.01 M HCl

The results are given in Table 2 below.

TABLE 2

|  | $HNO_3$ 1.0 M | $HNO_3$ 1.5 M | $HNO_3$ 2 M | $HNO_3$ 2.5 M | $HNO_3$ 3 M |
|---|---|---|---|---|---|
| 1st quart. | 95 | 70 | 17 | 14 | <1 |
| 2nd quart. | 5 | 30 | 78 | 75 | 3 |
| 3rd quart. |  | - | 5 | 11 | 45 |
| 4th quart. |  | - | - | - | 35 |

Thus by using the type of resin specified above a satisfactory distribution of the actinium is obtained starting from 2 M nitric acid solution.

EXAMPLE 3

In this example, the procedure followed in Example 1 is repeated but using, as cation exchange material, a highly cationic resin [bold] based on styrene and divinylbenzene (8% crosslinked) containing $-SO_3^-$ groups as ionic groups, the particle size was 100-200 mesh (75-150 micrometer) (AGMP50, BIORAD) and an HCl complexing agent solution having concentrations of from 2 to 4 M.

The results are given in Table 3 below.

TABLE 3

|  | HCl 2M | HCl 3M | HCl 4M |
|---|---|---|---|
| 1/4 | 100 | 5 | 1 |
| 2/4 | - | 68 | 15 |
| 3/4 | - | 27 | 53 |
| 4/4 | - | - | 28 |

Thus by using the type of resin specified above a satisfactory distribution of the actinium is obtained from 2.5-3.5 M hydrochloric acid solution.

EXAMPLE 4

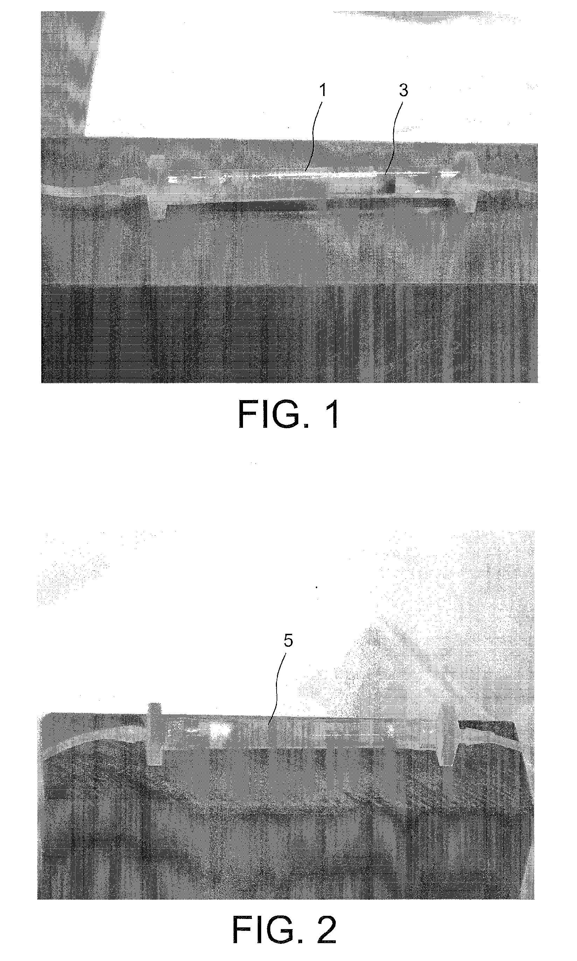

Two generators were loaded using a macroporous resin (matrix: styrene divinylbenzene, the functional group: $R-SO_3^-$) (AGMP50, BIORAD) the particle size was 100-200 mesh (75-150 micrometer) with 25 mCi actinium-225. The first one (generator referenced 1 on figure 1) was loaded using 2 M HCl as complexing agent. (Compare example 3: 2M HCl)(Geerlings et al., 1993, "The feasibility of $^{225}$Ac as a source of a-particles in radioimmunotherapy" Nucl Med Commun 14, 121-125). The second one (generator referenced 5 on figure 2) was loaded using 6,5 M $HNO_3$ as complex form agent. The elution yield, flow, $^{225}$Ac-breakthrough, labelling efficiency and visible changes of the matrix were recorded over 1 month period.

The elution yield of the generator A was during very first days approximately 90% from the theoretical maximum yield. After one week use the elution yield was decreased under 50% and remarkable higher pressure was required to push elution solution through the column. Simultaneously, labelling efficiency was reduced down 40 to 60%. After two weeks period the column was almost blocked and the elution yield was poor.

The elution yield of the generator B was approximately 90% from the theoretical maximum yield over one month. No changes in the flow were observed. The labelling efficiency was approximately the same (70%) over one month and no actinium-225 contamination was observed in the final product.

Both generators were photographed after one month (Generator A: Figure 1 and Generator B: figure 2. The arrows in the figures are indicating flow-direction). In the figure 1 (generator A) the strong black band (reference 3) on the top of the column is caused by strong local radiation. Otherwise in the figure 2 (generator) radiolyse damages are much less visible and they are well distributed over whole first half top part of the generator.

**Claims**

1.  Method of loading a mother radionuclide into a radionuclide generator which comprises a cation exchange material, the said method comprising a preliminary step which consists in passing over the said material a solution containing the mother radionuclide and a complexing agent for the said radionuclide, the said complexing agent being at an effective concentration such as to prevent the mother radionuclide from concentrating solely in one part of the volume of the cation exchange material, the cation exchange material being chosen in the group consisting of the macroporous resins, the resins having a crosslinking rate of from 1 to 12%.

2.  Method according to Claim 1, wherein the complexing agent is at a concentration which is effective to give a distribution coefficient $K_D$ between the solution and the cation exchange material such that $K_D$ is from 0.5 to 0.99.

3.  Method according to Claim 2, wherein $K_D$ is from 0.7 to 0.98.

4.  Method according to any one of Claims 1 to 3, further comprising at least one step of washing, which consists, after the preliminary step, in passing over the cation exchange material a solution containing at least one complexing agent for the said mother radionuclide.

5.  Method according to any one of Claims 1 to 4, further comprising, after the preliminary step or the optional washing step(s) at least one step consisting in passing over the said cation exchange material a solution containing an agent capable of destroying the complex formed between the mother radionuclide and its complexing agent.

6.  Method according to any one of the preceding claims, wherein the mother radionuclide is $^{225}$Ac.

7.  Method according to Claim 6, wherein the cation exchange material is a macroporous resin based on styrene and divinylbenzene bearing $-SO_3^-$ ionic groups.

8.  Method according to Claim 6 or 7, wherein the complexing agent is selected from nitric acid, hydrochloric acid and mixtures thereof.

9.  Method according to Claim 8, wherein the complexing agent is nitric acid.

10. Method according to Claim 9, wherein the nitric acid has a concentration greather than or equal to 1.5 M.

11. Method according to Claim 9, wherein the nitric acid has a concentration of from 5 to 7.5 M and the resin is macroporous resin.

FIG. 1

FIG. 2

**European Patent Office**

## PARTIAL EUROPEAN SEARCH REPORT

which under Rule 45 of the European Patent Convention
shall be considered, for the purposes of subsequent
proceedings, as the European search report

Application Number

EP 03 10 1480

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | GEERLINGS M W ET AL: "THE FEASIBILITY OF 225 AC AS A SOURCE OF ALPHA-PARTICLES IN RADIOIMMUNOTHERAPY" NUCLEAR MEDICINE COMMUNICATIONS, XX, XX, vol. 14, no. 121, 1993, pages 121-125, XP001079132 ISSN: 0143-3636 | 1-11 | A61K51/12 A61K51/06 G21G4/08 |
| Y | Paragraph "extraction of 225Ac and 213Bi from their 229Th and 225Ac sources" | 1-11 | |
| X | KASPERSEN F M ET AL: "CYTOTOXICITY OF 213BI- AND 225AC-IMMUNOCONJUGATES" NUCLEAR MEDICINE COMMUNICATIONS, XX, XX, vol. 16, 1995, pages 468-476, XP000673367 ISSN: 0143-3636 Paragraph "methods" | 1-9 | |

-/--

**TECHNICAL FIELDS SEARCHED (Int.Cl.7)**

A61K
G21G

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 24 November 2003 | Vadot, P |

EPO FORM 1503 03.82 (P04C07)

Reason for the limitation of the search:

Present claims 1-5 relate to an extremely large number of possible methods. Support within the meaning of Article 84 EPC and/or disclosure within the meaning of Article 83 EPC is to be found, however, for only a very small proportion of the methods claimed. In the present case, the claims so lack support, and the application so lacks disclosure, that a meaningful search over the whole of the claimed scope is impossible. Consequently, the search has been carried out for those parts of the claims which appear to be supported and disclosed, namely the examples.

**European Patent Office**

## PARTIAL EUROPEAN SEARCH REPORT

Application Number

EP 03 10 1480

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X,D | ZUCCHINI G L ET AL: "ISOTOPIC GENERATOR FOR 212PB AND 212BI" INTERNATIONAL JOURNAL OF NUCLEAR MEDICINE AND BIOLOGY, PERGAMON PRESS, OXFORD, GB, vol. 9, no. 1, 1982, pages 83-84, XP001030049 ISSN: 0047-0740 * the whole document * --- | 1-5 | |
| X,D | ATCHER R W ET AL: "AN IMPROVED GENERATOR FOR THE PRODUCTION OF 212PB AND 212BI FROM 224RA" APPLIED RADIATION AND ISOTOPES, INTERNATIONAL JOURNAL OF RADIATION APPLICATIONS AND INSTRUMENTATION, PART A, PERGAMON PRESS LTD, GB, vol. 39, no. 4, 1988, pages 283-286, XP001030045 ISSN: 0883-2889 Paragraph "material and methods" --- | 1-5 | |
| X | US 4 663 129 A (FRIEDMAN ARNOLD M ET AL) 5 May 1987 (1987-05-05) Paragraph "example" --- | 1-5 | TECHNICAL FIELDS SEARCHED (Int.Cl.7) |
| X | BEHR ET AL.: "High-linear energy transfer alpha vesrus low LET beta emitters in radioimmunotherapy of solid tumors: therapeutic efficacy and dose-limiting toxicity of 213Bi-versus 90Y-labeled CO17-1A Fab' fragments in a human colonic cancer model" CANCER RESEARCH, vol. 59, 1999, pages 2635-2643, XP002257157 Paragraph "Materials and methods", Radiolabeling --- | 1-8 | |

-/--

EPO FORM 1503 03.82 (P04C10)

**European Patent Office**     **PARTIAL EUROPEAN SEARCH REPORT**     Application Number

EP 03 10 1480

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| Y | WU ET AL.: "An improved generator for the production of 213Bi from 225Ac." RADIOCHIMICA ACTA, vol. 79, 1997, pages 141-144, XP008022994 Paragraph "experimental", Materials, Preparation of the 225Ac/213Bi generator --- | 1-11 | |
| X | EP 0 585 986 A (AKZO NV) 9 March 1994 (1994-03-09) Example 2 --- | 1-5 | |
| X | BOLL R A ET AL: "The Ac/Bi biomedical generator" TRANSACTIONS OF THE AMERICAN NUCLEAR SOCIETY, AMERICAN NUCLEAR SOCIETY, LA GRANGE PARK, IL, US, no. 77, 16 November 1997 (1997-11-16), pages 523-524, XP002084478 ISSN: 0003-018X Last paragraph --- | 1-7 | |
| X | BOLL ET AL.: "Optimizations of radiolabeling of immunoproteins with 213Bi" RADIOCHIMICA ACTA, vol. 79, 1997, pages 145-149, XP008023112 Paragraph "experimental ", Separation of Ac from Ra and preparation of 225Ac/213Bi generator ----- | 1-7 | **TECHNICAL FIELDS SEARCHED (Int.Cl.7)** |

EPO FORM 1503 03.82 (P04C10)

## ANNEX TO THE EUROPEAN SEARCH REPORT
## ON EUROPEAN PATENT APPLICATION NO.

EP 03 10 1480

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2003

| Patent document cited in search report | | Publication date | Patent family member(s) | | Publication date |
|---|---|---|---|---|---|
| US 4663129 | A | 05-05-1987 | NONE | | |
| EP 0585986 | A | 09-03-1994 | EP | 0585986 A1 | 09-03-1994 |
| | | | AT | 206622 T | 15-10-2001 |
| | | | AU | 676162 B2 | 06-03-1997 |
| | | | AU | 4215493 A | 03-02-1994 |
| | | | CA | 2100709 A1 | 28-01-1994 |
| | | | DE | 69330899 D1 | 15-11-2001 |
| | | | DE | 69330899 T2 | 25-04-2002 |
| | | | DK | 585986 T3 | 14-01-2002 |
| | | | ES | 2165361 T3 | 16-03-2002 |
| | | | FI | 933350 A | 28-01-1994 |
| | | | JP | 6157350 A | 03-06-1994 |
| | | | MX | 9304499 A1 | 29-04-1994 |
| | | | NO | 932687 A | 28-01-1994 |
| | | | NZ | 248235 A | 27-04-1995 |
| | | | PT | 585986 T | 28-03-2002 |
| | | | US | 6127527 A | 03-10-2000 |
| | | | US | 5641471 A | 24-06-1997 |
| | | | ZA | 9305052 A | 07-02-1994 |

EPO FORM P0459

For more details about this annex : see Official Journal of the European Patent Office, No. 12/82